# EUROPÄISCHE PATENTSCHRIFT

(11) **EP 0 975 347 B1**
(45) Veröffentlichungstag und Bekanntmachung des Hinweises auf die Patenterteilung: **20.02.2008**
(21) Anmeldenummer: 98913584.3
(22) Anmeldetag: 24.02.1998
(51) Int. Cl.: A61K 31/557, A61P 11/00

(54) **SYNERGISTISCHE KOMBINATION VON PDE-HEMMERN UND ADENYLATCYCLASE-AGONISTEN BZW. GUANYLCYCLYSE-AGONISTEN**
SYNERGISTIC COMBINATION OF PDE INHIBITORS AND ADENYLATE CYCLASE AGONISTS OR GUANYL CYCLYSE AGONISTS
COMBINAISON SYNERGIQUE D'INHIBITEURS DE LA PHOSPHODIESTERASE ET D'AGONISTES DE LA CYCLASE D'ADENYLATE OU D'AGONISTES DE LA CYCLYSE GUANYLIQUE

(30) Priorität: 28.02.1997 DE 19708049
(43) Veröffentlichungstag der Anmeldung: 02.02.2000
(73) Patentinhaber: Nycomed GmbH, 78467 Konstanz (DE)
(72) Erfinder: BEUME, Rolf, D-78465 Konstanz (DE); FLOCKERZI, Dieter, D-78476 Allensbach (DE); BELLER, Klaus-Dieter, D-79341 Kenzingen (DE); GRIMMINGER, Friedrich, D-35510 Butzbach (DE); SUTTORP, Norbert, D-35578 Wetzlar (DE); SCHUDT, Christian, D-78462 Konstanz (DE)
(86) Internationale Anmeldenummer: PCT/EP1998/001047
(87) Internationale Veröffentlichungsnummer: WO 1998/037894

(56) Entgegenhaltungen:
- WO-A-96/06612
- US-A- 4 721 729
- BUERKE M. ET AL: "Synergistic platelet inhibitory effect of the phosphodiesterase inhibitor Piroximone and Iloprost" AGENTS ACTIONS, 1992, 37/SUPPL. (71-77), SWITZERLAND, XP002071735
- O'GRADY J ET AL: "A CHEMICALLY STABLE ANALOG 9-BETA METHYLCARBACYCLIN WITH SIMILAR EFFECTS TO EPOPROSTENOL PROSTACYCLIN PROSTAGLANDIN I-2 IN MAN" BR J CLIN PHARMACOL, 18 (6). 1984 (RECD. 1985). 921-934., XP002071736
- CRUTCHLEY D J ET AL: "Effects of Prostacyclin Analogs on the Synthesis of Tissue Factor, Tumor Necrosis Factor-alpha and Interleukin-1-beta in Human Monocytic THP-1 Cells" JOURNAL OF PHARMACOLOGY AND EXPERIMENTAL THERAPEUTICS, 271 (1). 1994. 446-451., XP002071737
- CRUTCHLEY D J ET AL: "PROSTACYCLIN ANALOGUES INHIBIT TISSUE FACTOR EXPRESSION IN THE HUMAN MONOCYTIC CELL LINE THP-1 VIA A CYCLIC AMP-DEPENDENT MECHANISM" ARTERIOSCLER THROMB, 12 (6). 1992. 664-670., XP002071738
- RIVA C M ET AL: "ILOPROST INHIBITS NEUTROPHIL-INDUCED LUNG INJURY AND NEUTROPHIL ADHERENCE TO ENDOTHELIAL MONOLAYERS" AM J RESPIR CELL MOL BIOL, 3 (4). 1990. 301-310., XP002071739

## Beschreibung

### Anwendungsgebiet der Erfindung

Die Erfindung betrifft die Kombination bestimmter bekannter Wirkstoffe zu therapeutischen Zwecken.

### Bekannter technischer Hinter₋grund

Bei den in der erfindungsgemäßen Kombination angewandten Substanzen handelt es sich um bekannte Wirkstoffe aus der Klasse der PDE4-Hemmer sowie aus der Klasse der Adenylatcyclase-Agonisten. Ihre kombinierte Anwendung im erfindungsgemäßen Sinn zu therapeutischen Zwecken ist im Stand der Technik noch nicht beschrieben.

### Beschreibung der Erfindung

Gegenstand der Erfindung ist die kombinierte Anwendung von PDE4-Hemmern einerseits sowie Adenylatcyclase-Agonisten andererseits bei der Behandlung von akuter oder chronischer Obstruktion der Bronchien und/oder chronischer Entzündung der Bronchien.

PDE-Hemmer im Sinne der vorliegenden Erfindung sind solche Verbindungen, die durch Hemmung der Phosphodiesterasen den Abbau von zyklischem AMP (cAMP) verlangsamen, was zu einer relativen Steigerung der intrazellulären Konzentration von cAMP führen kann.

Beispielhaft seien solche PDE-Hemmer genannt, wie sie in den folgenden Patentanmeldungen und Patenten beschrieben bzw. beansprucht sind: DE2315801 und WO9501338.

Als beispielhafte PDE4-Hemmer seien genannt IBUDILAST und N-(3,5-Dichlorpyrid-4-yl)-3-cyclopropylmethoxy-4-difluormethoxybenzamid.

Adenylatcyclase-Agonisten (AC-Agonisten) im Sinne der vorliegenden Erfindung sind solche Verbindungen, die über das Enzym Adenylatcyclase die Synthese von cAMP beschleunigen, was zu einer relativen Steigerung der intrazellulären Konzentration von cAMP führt.

Als AC-Agonisten seien neben den spezifischen Prostaglandinen beispielsweise Adenosin, endothelspezifische Wachstumsfaktoren (VEGF) und β-Agonisten, vor allem β₂-Sympathomimetika, genannt. Unter den β₂-Sympathomimetika seien vor allem solche selektiv wirkende Substanzen genannt, die nur eine geringe kardiale Wirkung haben und daher auch in der Therapie des Asthma bronchiale eingesetzt werden. Als entsprechende β₂-Sympathomimetika seien beispielsweise genannt: Salbutamol, Tulobuterol, Terbutalin, Carbuterol, Pirbuterol, Isoxsuprin, Reproterol, Clenbuterol, Fenoterol, Bamethan, Hexoprenalin, Formoterol, Salmeterol, Picumeterol, Rimiterol, Procaterol, Bambuterol, Bitolterol, Mabuterol, Clorprenalin, Isoetarin, Etanterol, Imoxiterol, Naminterol, Salmefamol und Zinterol.

Folgende Aspekte sind von der vorliegenden Erfindung umfasst:

PDE4-Hemmer kombiniert mit einem β2-Sympathomimetikum zur Anwendung bei der therapeutischen Behandlung von akuter oder chronischer Obstruktion der Bronchien und/oder chronischen Entzündungserkrankungen der Bronchien, dadurch gekennzeichnet, dass ein PDE4-Hemmer verwendet wird, der ausgewählt ist aus der Gruppe bestehend aus IBUDILAST und N-(3,5-Dichlorpyrid-4-yl)-3-cyclopropylmethoxy-4-difluormethoxybenzamid, und dass das β2-Sympathomimetikum ausgewählt ist aus der Gruppe bestehend aus Salbutamol, Tulobuterol, Terbutalin, Carbuterol, Pirbuterol, Isoxsuprin, Reproterol, Clenbuterol, Fenoterol, Bamethan, Hexoprenalin, Formoterol, Salmeterol, Picumeterol, Rimiterol, Procaterol, Bambuterol, Bitolterol, Mabuterol, Clorprenalin, Isoetarin, Etanterol, Imoxiterol, Naminterol, Salmefamol und Zinterol.

Verwendung eines PDE4-Hemmers kombiniert mit einem β2-Sympathomimetikum zur Herstellung eines Arzneimittels zur Anwendung bei der therapeutischen Behandlung von akuter oder chronischer Obstruktion der Bronchien und/oder chronischen Entzündungserkrankungen der Bronchien, dadurch gekennzeichnet, dass ein PDE4-Hemmer verwendet wird, der ausgewählt ist aus der Gruppe bestehend aus IBUDILAST und N-(3,5-Dichlorpyrid-4-yl)-3-cyclopropylmethoxy-4-difluormethoxybenzamid, und dass das β2-Sympathomimetikum ausgewählt ist aus der Gruppe bestehend aus Salbutamol, Tulobuterol, Terbutalin, Carbuterol, Pirbuterol, Isoxsuprin, Reproterol, Clenbuterol, Fenoterol, Bamethan, Hexoprenalin, Formoterol, Salmeterol, Picumeterol, Rimiterol, Procaterol, Bambuterol, Bitolterol, Mabuterol, Clorprenalin, Isoetarin, Etanterol, Imoxiterol, Naminterol, Salmefamol und Zinterol.

Kombinationsarzneimittel zur Anwendung bei der therapeutischen Behandlung von akuter oder chronischer Obstruktion der Bronchien und/oder chronischen Entzündungserkrankungen der Bronchien enthaltend einen PDE4-Hemmer kombiniert mit einem β2-Sympathomimetikum, dadurch gekennzeichnet, dass ein PDE4-Hemmer verwendet wird, der ausgewählt ist aus der Gruppe bestehend aus IBUDILAST und N-(3,5-Dichlorpyrid-4-yl)-3-cyclopropylmethoxy-4-difluormethoxybenzamid, und dass das β2-Sympathomimetikum ausgewählt ist aus der Gruppe bestehend aus Salbutamol, Tulobuterol, Terbutalin, Carbuterol, Pirbuterol, Isoxsuprin, Reproterol, Clenbuterol, Fenoterol, Bamethan, Hexoprenalin, Formoterol, Salmeterol, Picumeterol, Rimiterol, Procaterol, Bambuterol, Bitolterol, Mabuterol, Clorprenalin, Isoetarin, Etanterol, Imoxiterol, Naminterol, Salmefamol und Zinterol.

Eine Beschleunigung der Synthese (durch den AC-Agonisten) kann ebenso wie eine Hemmung des Abbaus (durch den PDE-Hemmer) zu einer relativen Steigerung der intrazellulären Konzentration von cAMP führen.

Die daraus folgenden biologischen Effekte der Kombination sind an zellulären Modellsystemen nicht zwangsläufig additiv oder sogar überadditiv. Überraschenderweise wurde an der isoliert perfundierten Lunge des Kaninchens ein deutlich überadditiver synergistischer Effekt auf den experimentellen pulmonalen Hochdruck beobachtet.

Die unerwartete, überadditive Steigerung der Wirkung eines AC-Agonisten auf die Höhe und Dauer des pulmonalen Blutdrucks durch die gleichzeitige Gabe eines PDE-Hemmers zeigt eine besondere Eignung für die Behandlung von Krankheitszuständen wie z.B. pulmonale Hypertonie. Darüber hinaus kann von einer Dauerbehandlung mit einer solchen Kombination eine positive Beeinflussung der chronischen Veränderungen des Bronchialsystems ("Remodeling") erwartet werden. Dies gilt für sämtliche Krankheitszustände, die durch eine chronische Angiopathie charakterisiert sind.

Durch die erfindungsgemäße Kombination von PDE-Hemmer und AC-Agonist können die Einzelkomponenten in Konzentrationen verwendet werden, die alleine wenig oder gar nicht wirksam sind. Hierdurch werden Nebenwirkungen der Einzelkomponenten, die bei den eigenwirksamen Konzentrationen von PDE-Hemmer oder AC-Agonist bei alleiniger Gabe auftreten würden, durch die niedrige Konzentration in der Kombination vermieden.

Darüber hinaus sind die Effekte der Kombination überraschenderweise auch noch deutlich länger anhaltend als die Effekte der Einzelkomponenten.

Als Krankheitszustände, die durch die erfindungsgemäße Kombination behandelt werden können, seien beispielsweise genannt: chronische Obstruktion von Atemwegen (bei Resistenz oder Überempfindlichkeit gegen relaxierende AC-Agonisten - z.B. zur Bronchorelaxation - kann deren Wirkung bei kleineren Konzentrationen durch die zusätzliche Gabe von geeigneten PDE-Hemmem verstärkt werden), irreversible Obstruktionen von Bronchien (als Folge von Entzündungserkrankungen wie z.B. Asthma werden durch Proliferationsreize verschiedene Zellen zum Wachstum angeregt, was zur chronischen Verengung der Gefäße und der Bronchien führt; durch die erfindungsgemäße Kombination wird das Zellwachstum verlangsamt und die als "irreversibel" bekannten Obstruktionen in Bronchien können reduziert werden); des weiteren können Entzündungen (die transpulmonale Lymphozytenkinetik und der Granulozyteneinstrom) wirkungsvoll verhindert werden.

"Kombinierte Anwendung" im Sinne der vorliegenden Erfindung ist so zu verstehen, daß die Einzelkomponenten in an sich bekannter und gewohnter Weise gleichzeitig (in Form eines Kombinationsarzneimittels), mehr oder weniger zeitgleich (aus getrennten Verpackungseinheiten) oder nacheinander (direkt nacheinander oder aber auch mit größerem zeitlichen Abstand) verabfolgt werden können.

Bei mehr oder weniger zeitgleicher Verabfolgung der Einzelkomponenten aus getrennten Verpackungseinheiten und bei der nacheinander erfolgenden Applikation der Einzelkomponenten kann gewünschtenfalls eine unterschiedliche Applikationsform gewählt werden. Beispielsweise kann die eine Komponente inhalativ verabreicht werden, während die andere Komponente durch Infusion verabfolgt wird.

Die Dosierung der Wirkstoffe erfolgt in einer für die Dosierung der Einzelkomponenten üblichen Größenordnung, wobei aufgrund der sich gegenseitig positiv beeinflussenden und verstärkenden Einzelwirkungen die jeweiligen Dosierungen bei der kombinierten Gabe der Wirkstoffe gegenüber der Norm verringert werden können.

## Patentansprüche

1. PDE4-Hemmer kombiniert mit einem β2-Sympathomimetikum zur Anwendung bei der therapeutischen Behandlung von akuter oder chronischer Obstruktion der Bronchien und/oder chronischen Entzündungserkrankungen der Bronchien, **dadurch gekennzeichnet, dass** ein PDE4-Hemmer verwendet wird, der ausgewählt ist aus der Gruppe bestehend aus IBUDILAST und N-(3,5-Dichlorpyrid-4-yl)-3-cyclopropylmethoxy-4-difluormethoxybenzamid, und dass das β2-Sympathomimetikum ausgewählt ist aus der Gruppe bestehend aus Salbutamol, Tulobuterol, Terbutalin, Carbuterol, Pirbuterol, Isoxsuprin, Reproterol, Clenbuterol, Fenoterol, Bamethan, Hexoprenalin, Formoterol, Salmeterol, Picumeterol, Rimiterol, Procaterol, Bambuterol, Bitolterol, Mabuterol, Clorprenalin, Isoetarin, Etanterol, Imoxiterol, Naminterol, Salmefamol und Zinterol.

2. Verwendung eines PDE4-Hemmers kombiniert mit einem β2-Sympathomimetikum zur Herstellung eines Arzneimittels zur Anwendung bei der therapeutischen Behandlung von akuter oder chronischer Obstruktion der Bronchien und/oder chronischen Entzündungserkrankungen der Bronchien, **dadurch gekennzeichnet, dass** ein PDE4-Hemmer verwendet wird, der ausgewählt ist aus der Gruppe bestehend aus IBUDILAST und N-(3,5-Dichlorpyrid-4-yl)-3-cyclopropylmethoxy-4-difluormethoxybenzamid, und dass das β2-Sympathomimetikum ausgewählt ist aus der Gruppe bestehend aus Salbutamol, Tulobuterol, Terbutalin, Carbuterol, Pirbuterol, Isoxsuprin, Reproterol, Clenbuterol, Fenoterol, Bamethan, Hexoprenatin, Formoterol, Salmeterol, Picumeterol, Rimiterol, Procaterol, Bambuterol, Bitolterol, Mabuterol, Clorprenalin, Isoetarin, Etanterol, Imoxiterol, Naminterol, Salmefamol und Zinterol.

3. Kombinationsarzneimittel zur Anwendung bei der therapeutischen Behandlung von akuter oder chronischer Obstruktion der Bronchien und/oder chronischen Entzündungserkrankungen der Bronchien enthaltend einen PDE4-Hemmer kombiniert mit einem β2-Sympathomimetikum, **dadurch gekennzeichnet, dass** ein PDE4-Hemmer verwendet wird, der ausgewählt ist aus der Gruppe bestehend aus IBUDILAST und N-(3,5-Dichlorpyrid-4-yl)-3-cyclopropylmethoxy-4-difluormethoxybenzamid, und dass das β2-Sympathomimetikum ausgewählt ist aus der Gruppe bestehend aus Salbutamol, Tulobuterol, Terbutalin, Carbuterol, Pirbuterol, Isoxsuprin, Reproterol, Clenbuterol, Fenoterol, Bamethan, Hexoprenalin, Formoterol, Salmeterol, Picumeterol, Rimiterol, Procaterol, Bambuterol, Bitolterol, Mabuterol, Clorprenalin, Isoetarin, Etanterol, Imoxiterol, Naminterol, Salmefamol und Zinterol.

## Claims

1. PDE4 inhibitor combined with a β2-sympathomimetic for use in the therapeutic treatment of acute or chronic obstruction of bronchi and/or acute or chronic inflammatory diseases of the bronchi, **characterized in that** a PDE4 inhibitor is used, selected from the group consisting if IBUDILAST and N-(3,5-dichloropyrid-4-yl)-3-cyclopropylmethoxy-4-difluoromethoxybenzamide, and **in that** the β2-sympathomimetic is selected from the group consisting of salbutamol, tulobuterol, terbutalin, carbuterol, pirbuterol, isoxsuprine, reproterol, clenbuterol, fenoterol, bamethan, hexoprenaline, formoterol, salmeterol, picumeterol, rimiterol, procaterol, bambuterol, bitolterol, mabuterol, clorprenaline, isoetarine, etanterol, imoxiterol, naminterol, salmefamol and zinterol.

2. Use of a PDE4 inhibitor combined with a β2-sympathomimetic for the preparation of a medicament for use in the therapeutic treatment of acute or chronic obstruction of bronchi and/or acute or chronic inflammatory diseases of the bronchi, **characterized in that** a PDE4 inhibitor is used, selected from the group consisting if IBUDILAST and N-(3,5-dichloropyrid-4-yl)-3-cyclopropylmethoxy-4-difluoromethoxybenzamide, and **in that** the β2-sympathomimetic is selected from the group consisting of salbutamol, tulobuterol, terbutalin, carbuterol, pirbuterol, isoxsuprine, reproterol, clenbuterol, fenoterol, bamethan, hexoprenaline, formoterol, salmeterol, picumeterol, rimiterol, procaterol, bambuterol, bitolterol, mabuterol, clorprenaline, isoetarine, etanterol, imoxiterol, naminterol, salmefamol and zinterol.

3. Combination medicament for use in the therapeutic treatment of acute or chronic obstruction of bronchi and/or acute or chronic inflammatory diseases of the bronchi, comprising a PDE4 inhibitor combined with a β2-sympathomimetic, **characterized in that** a PDE4 inhibitor is used, selected from the group consisting of IBUDILAST and N-(3,5-dichloropyrid-4-yl)-3-cyclopropylmethoxy-4-difluoromethoxybenzamide, and **in that** the β2-sympathomimetic is selected from the group consisting of salbutamol, tulobuterol, terbutalin, carbuterol, pirbuterol, isoxsuprine, reproterol, clenbuterol, fenoterol, bamethan, hexoprenaline, formoterol, salmeterol, picumeterol, rimiterol, procaterol, bambuterol, bitolterol, mabuterol, clorprenaline, isoetarine, etanterol, imoxiterol, naminterol, salmefamol and zinterol.

## Revendications

1. Inhibiteur de la PDE4, combiné à un β2-mimétique, pour utilisation lors du traitement thérapeutique d'une obstruction aiguë ou chronique des bronches et/ou de maladies inflammatoires chroniques des bronches, **caractérisé en ce qu'**on utilise un inhibiteur de la PDE4 qui est choisi dans le groupe constitué de l'IBUDILAST et du N-(3,5-dichloropyrid-4-yl)-3-cyclopropylméthoxy-4-difluorométhoxybenzamide, et **en ce que** le β2-mimétique est choisi dans le groupe constitué du salbutamol, du tulobutérol, de la terbutaline, du carbutérol, du pirbutérol, de l'isoxsuprine, du réprotérol, du clenbutérol, du fénotérol, du baméthan, de l'hexoprénaline, du formotérol, du salmétérol, du picumétérol, du rimitérol, du procatérol, du bambutérol, du bitoltérol, du mabutérol, de la clorprénaline, de l'isoétarine, de l'étantérol, de l'imoxitérol, du namintérol, du salméfamol et du zintérol.

2. Utilisation d'un inhibiteur de la PDE4, combiné à un β2-mimétique, pour préparer un médicament destiné à une utilisation lors du traitement thérapeutique d'une obstruction aiguë ou chronique des bronches et/ou de maladies inflammatoires chroniques des bronches, **caractérisée en ce qu'**on utilise un inhibiteur de la PDE4 qui est choisi dans le groupe constitué de l'IBUDILAST et du N-(3,5-dichloropyrid-4-yl)-3-cyclopropylméthoxy-4-difluorométhoxybenzamide, et **en ce que** le β2-mimétique est choisi dans le groupe constitué du salbutamol, du tulobutérol, de la terbutaline, du carbutérol, du pirbutérol, de l'isoxsuprine, du réprotérol, du clenbutérol, du fénotérol, du baméthan, de l'hexoprénaline, du formotérol, du salmétérol, du picumétérol, du rimitérol, du procatérol, du bambutérol, du bitoltérol, du mabutérol, de la clorprénaline, de l'isoétarine, de l'étantérol, de l'imoxitérol, du namintérol, du salméfamol et du zintérol.

3. Médicament en association, pour une utilisation lors du traitement thérapeutique d'une obstruction aiguë ou chronique des bronches et/ou de maladies inflammatoires chroniques des bronches, contenant un inhibiteur de la PDE4 combiné à un β2-mimétique, **caractérisé en ce qu'**on utilise un inhibiteur de la PDE4 qui est choisi dans le groupe constitué de l'IBUDILAST et du N-(3,5-dichloropyrid-4-yl)-3-cyclopropylméthoxy-4-difluorométhoxybenzamide, et **en ce que** le β2-mimétique est choisi dans le groupe constitué du salbutamol, du tulobutérol, de la terbutaline, du carbutérol, du pirbutérol, de l'isoxsuprine, du réprotérol, du clenbutérol, du fénotérol, du baméthan, de l'hexoprénaline, du formotérol, du salmétérol, du picumétérol, du rimitérol, du procatérol, du bambutérol, du bitoltérol, du mabutérol, de la clorprénaline, de l'isoétarine, de l'étantérol, de l'imoxitérol, du namintérol, du salméfamol et du zintérol.
